# EUROPEAN PATENT APPLICATION

(11) **EP 2 074 889 A1**
(43) Date of publication of application: **01.07.2009**
(21) Application number: 07829545.8
(22) Date of filing: 11.10.2007
(51) Int. Cl.: A23K 1/16, A23K 1/18, A61K 31/7028, A61K 31/7032, A61K 35/74, A61K 36/06, A61P 31/04, C07H 15/04

(54) **ANIMAL FEED ADDITIVE AND ANIMAL FEED**

(30) Priority: 17.10.2006 JP 2006282697; 31.05.2007 JP 2007144393
(71) Applicant: Idemitsu Kosan Co., Ltd., Chiyoda-ku Tokyo 100-8321 (JP)
(72) Inventor: ITO, Shinji, Sodegaura-shi Chiba 2990293 (JP); KOBAYASHI, Yasuo, Sapporo-shi Hokkaido 0600809 (JP); SUZUKI, Motoshi, . (JP); SUZUKI, Kuniki, Ichihara-shi, Chiba 2900056, (JP)
(74) Representative: Vossius & Partner
(86) International application number: PCT/JP2007/069807
(87) International publication number: WO 2008/047658

(57) **Abstract**

To provide safe and easy means for preventing or treating diseases of birds and mammals, in particular, livestock. In particular, to provide means for preventing or treating an infectious disease caused by a Gram-positive bacterium. In addition, to improve fermentation in the rumen of a ruminant animal, to contribute to suppression of the generation of greenhouse gas, and to increase the feed efficiency. Mannosylerythritol lipids (MEL) and/or rhamnolipids are given to birds or mammals.

## Description

### [Technical Field]

The present invention relates to feed additives and feeds containing glycolipids, and methods of breeding birds and mammals using the same.

### [Background Art]

Infectious diseases of livestock decrease the weight of the livestock and cause various symptoms, resulting in a significant decrease in a commercial value of the livestock. For example, *Staphylococcus aureus* is a bacterium that causes : mastitis, subcutaneous tumor, and pyemia of cow, sheep, and goat; rash of horse; arthritis, dermatitis, and septicemia of pigs and chickens . Meanwhile, *Streptococcus suis* is a bacterium that causes meningitis, septicemia, endocarditis, and arthritis of pig, while *Streptococcus bovis* is a bacterium that causes bloat of cow.

The fact that addition of a small amount of an antibiotic to a livestock feed promotes the growth of livestock was discovered in 1940's, and since then, addition of an antibiotic to a livestock feed has been widely performed to promote the growth of livestock or to prevent a disease. An antibiotic is considered to have abilities to prevent infection of a pathogenic bacterium in livestock, to improve metabolism, and to suppress amplification of a harmful enteric bacterium, resulting in prevention of a disease and promotion of growth, but the details remain unknown. Meanwhile, addition of an antibiotic to a feed may spread the antibiotic to the natural environment, and appearance of an antibiotic-resistant bacterium has become a huge issue in the livestock industry. For example, it has been reported that a typical antibiotic-resistant bacterium, MRSA (methicillin-resistant *Staphylococcus aureus)* was discovered in livestock such as horse.
Under such circumstances, in recent years, addition of an antibiotic to a feed is heavily regulated. For example, in Europe, feeds containing antibiotics were totally banned by 2006, and in Japan, the number of antibiotics that can be used is gradually decreasing. Moreover, manufacturers demand alternatives to antibiotics to solve the above-mentioned problems.

Because of such movement of alternatives to antibiotics, there are some attempts to use polypeptides such as nisin produced by lactic acid bacteria and iturin produced by *Bacillus* bacteria. Meanwhile, sucrose esters, which are glycolipids to be added to canned coffee or the like as emulsifiers, which are expected to have antibiotic activities to *Bacillus* bacteria or the like, are added to feed.

A ruminant animal such as cow or sheep lives by a fermentation product obtained by digesting/fermenting a feed using a microorganism in the rumen. Therefore, methane generation from the rumen leads to loss of the energy efficiency of the feed. Moreover, methane is a greenhouse gas that affects global warming, therefore, it is important to reduce the methane generation in the rumen of a ruminant animal.
A methane-producing bacterium in a rumen produces methane by reducing carbon dioxide using hydrogen. The contribution ratio of methane to global warming is the second highest after carbon dioxide, and methane released from ruminant animals accounts for 15 to 20% of the total methane release.

Ionophores such as an antibiotic monensin are widely used in feeds for ruminant animals. Monensin has an effect of selectively suppressing microorganisms in a rumen, resulting in reduction in the generation of methane and promotion of the generation of propionic acid. Propionic acid has a high ATP generation efficiency compared with other volatile fatty acids, therefore, the promotion of the generation of propionic acid improves the feed efficiency.

From such circumstances, it has been desired to develop an alternative to monensin or the like to be added to feeds for ruminant animals. In order to obtain the alternative, studies have been made on a plant extraction oil (Non-Patent Document 1), an anti-lactic acid-producing bacterium vaccine (Non-Patent Document 2), anti-lactic acid-producing bacterium hen egg antibody (Non-Patent Document 3), etc. However, those technologies have problems, for example, the effect is not stabilized and registration of feeds containing these materials is not accepted. Therefore, those technologies have not been put to practical use.

On the other hand, glycolipids represented by mannosylerythritol lipids (MEL) and rhamnolipids (RL) have various properties such as surfactant activities and are used for various purposes as described below. For example, there are known a technology for improving the gene transduction efficiencies using a liposome containing MEL (Patent Document 1), a method of inhibiting the formation of a liposome containing a drug-resistant gene or the like using MEL to decrease the generation of a drug-resistant bacterium or the like (Patent Document 2), a technology using MEL as an active ingredient of an anti-inflammatory agent and an antiallergic agent (Patent Document 3), etc. In addition, there are known a technology for improving the water-absorbing property of a natural fiber using rhamnolipids (Patent Document 4), a technology for separating a harmful useful organic compound from an untreated product containing the harmful useful organic compound using rhamnolipids (Patent Document 5), a technology for preventing the aggregation and coalescence of ice by preparing ice slurry for high-density thermal regulating transportation using rhamnolipids (Patent Document 6), etc. Note that, some reports on antibiotic properties of MEL and rhamnolipids have been made (Non-Patent Documents 4 and 5), but antibiotic properties to bacteria causing infectious diseases of livestock have not been studied, and there is no example of applications of MEL and rhamnolipids to the livestock industry.

[Patent Document 1] JP 2006-174727 A
[Patent Document 2] JP 2006-158387 A
[Patent Document 3] JP 2005-68015 A
[Patent Document 4] JP 2002-105854 A
[Patent Document 5] JP 2001-327803 A
[Patent Document 6] JP 2001-131538 A
[Non-patent Document 1] Benchaar et al., Can.J.Anim.Sci. 86, 91-96(2006)
[Non-patent Document 2] Shu et al., FEMS Immunuology & Medical Microbiology, 26(2), 153-158(1999)
[Non-patent Document 3] DiLorenzo et al., J.Anim.Sci., 84,2178-2185(2006)
[Non-patent Document 4] Fat.Sci.Technol., 91, 363-366, 1989
[Non patent Document 5] Biotechnol., 29, 91-96, 1993

### [Disclosure of the Invention]

An object of the present invention is to provide a safe and easy means for preventing or treating diseases of birds and mammals, in particular, livestock. In particular, an object of the present invention is to provide a means for preventing or treating an infectious disease caused by a Gram-positive bacterium.
Another object of the present invention is to improve fermentation in the rumen of a ruminant animal, to contribute to suppression of the generation of greenhouse gases, and to increase the feed efficiency.

The inventors of the present invention have made extensive studies to achieve the above-mentioned objects, and as a result, the inventors have found out that glycolipids such as mannosylerythritol lipids (MEL) and rhamnolipids have antibiotic activities to Gram-positive bacteria that causes infectious diseases of livestock, thus accomplished the present invention. Moreover, they have found out that glycolipids such as mannosylerythritol lipids (MEL) and rhamnolipids suppress the generation of methane and promote the generation of propionic acid in the rumen, thus accomplished the present invention.

That is, the present invention is as follows:
(1) a feed additive for birds and mammals comprising mannosylerythritol lipids and/or rhamnolipids;
(2) the feed additive according to Item (1), which is for livestock;
(3) the feed additive according to Item (2), wherein the livestock is chicken, pig, or cow;
(4) the feed additive according to Item (1), wherein for a ruminant animal;
(5) the feed additive according to any one of Items (1) to (4), wherein the mannosylerythritol lipids are obtained from a yeast belonging to the genus *Pseudozyma;*
(6) the feed additive according to any one of Items (1) to (5), wherein the rhamnolipids are obtained from a bacterium belonging to the genus *Pseudomonas;*
(7) the feed additive according to any one of Items (1) to (6), which is for prevention or treatment of a disease;
(8) the feed additive according to Item (7), wherein the disease is an infectious disease caused by a Gram-positive bacterium;
(9) the feed additive according to Item (8), in which the Gram-positive bacterium is a bacterium belonging to the genus *Staphylococcus* or *Streptococcus;*
(10) the feed additive according to Item (9), wherein the Gram-positive bacterium is *Staphylococcus aureus, Staphylococcus epidermidis, Streptococcus suis, or Streptococcus bovis;*
(11) a feed comprising the feed additive according to any one of Items (1) to (10); and
(12) a method of breeding birds or mammals, comprising giving the feed according to Item (11) to birds or mammals.

### [Brief Description of the Drawings]

[Fig. 1] Figs. 1 show effects of RL and MEL on gas generation amounts and compositions in a rumen.
[Fig. 2] Figs. 2 show effects of RL and MEL on concentrations and ratios of volatile fatty acids.

### [Best Modes for carrying out the Invention]

A feed additive of the present invention is characterized by comprising mannosylerythritol lipids (MEL) and/or rhamnolipids (RL).

MEL is one of glycolipid-type biosurfactants and has a structure including mannose, erythritol, and a fatty acid, and it is represented by the following general formula (1).

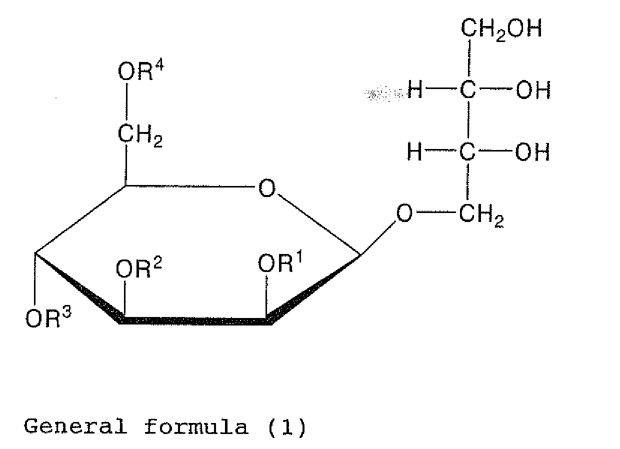

In general formula (1), R¹ and R² are each independently aliphatic acyl groups having 3 to 25 carbon atoms. In particular, R¹ and R² are preferably each independently aliphatic acyl groups having 5 to 14 carbon atoms. In particular, R¹ and R² are preferably each independently aliphatic acyl groups having 5 to 13 carbon atoms. Those aliphatic acyl groups may be linear or branched, and may be saturated or unsaturated. On the other hand, one of R³ and R⁴ is an acetyl group and the other is hydrogen, or both of R³ and R⁴ are acetyl groups.
MEL where both of R³ and R⁴ are acetyl groups is referred to as MEL-A, MEL where R³ is hydrogen and R⁴ is an acetyl group is referred to as MEL-B, and MEL where R³ is an acetyl group and R⁴ is hydrogen is referred to as MEL-C.
Meanwhile, a feed additive of the present invention may comprise one kind of MEL or plural kinds of MEL.

MEL to be used in the present invention can be obtained by culturing a microorganism such as a fungus, in particular, yeast. For example, there may be used yeasts belonging to the genera *Pseudozyma , Candida, and Kurtzmanomyces.* In addition, *Shizonella melanogramma* may be used. Of those, yeast belonging to the genus *Pseudozyma* is preferably used. Examples of the yeasts belonging to the genus *Pseudozyma* include *Pseudozyma aphidis* and *Pseudozyma antarctica*. Specifically, there may be used *Pseudozyma aphidis* NBRC 10182 strain, *Pseudozyma antarctica* NBRC 10260 strain, and *Pseudozyma Antarctica* NBRC 10736 strain, for example.
NBRC 10182 strain, NBRC 10260 strain, and NBRC 10736 strain are registered in the department of NITE Biological Resource Center (NBRC) in National Institute of Technology and Evaluation.
Meanwhile, the MEL may be synthesized or may be a commercially available product.

Rhamnolipid is one of glycolipid-type biosurfactants and has a structure including rhamnose and a fatty acid. Although rhamnolipids to be used in the present invention is not particularly limited, it may have the structure represented by the following general formula (2) or general formula (3).

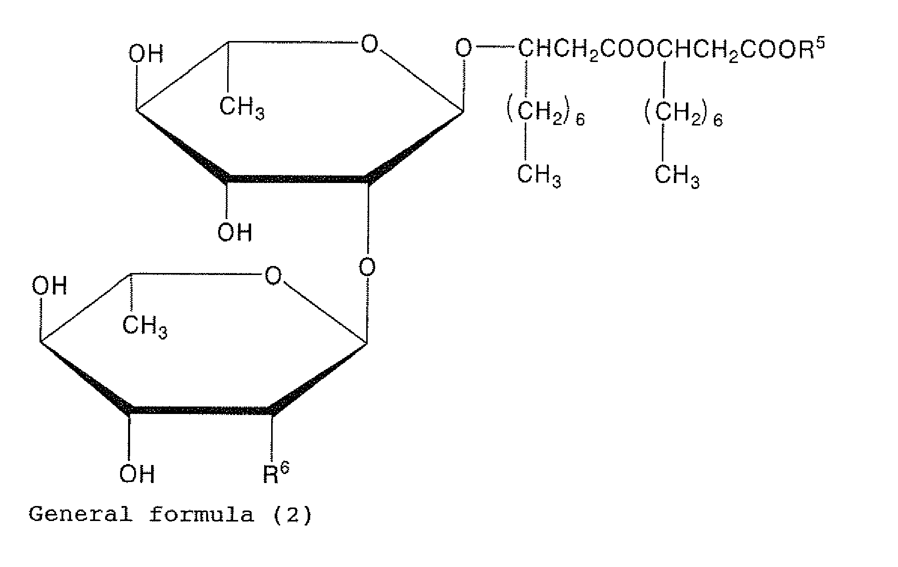

In general formula (2), R⁵ represents a hydrogen atom, -CH₂-[CH(OH)]ₘ-CH₂(OH), -(XO)ₙH, or an alkyl, alkenyl, aliphatic acyl group having 1 to 36 carbon atoms. In general formula (2), the alkyl and alkenyl groups may be linear or branched, and the aliphatic acyl group may be linear or branched, and may be saturated or unsaturated. Meanwhile, m is an integer of 0 to 8, and X represents at least one of ethylene, propylene, and butylene, and n is an integer of 1 to 1,000. R⁶ is a hydrogen atom or a 2-decenoyl group. R⁵ and R⁶ are independent from each other.

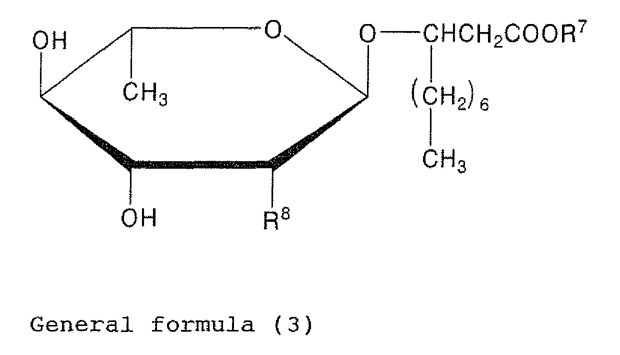

In general formula (3), R⁷ represents a hydrogen atom, -CH₂-[CH(OH)]ₘ-CH₂(OH), -(XO)ₙH, or an alkyl, alkenyl, aliphatic acyl group having 1 to 36 carbon atoms . In general formula (3), the alkyl and alkenyl groups may be linear or branched, and the aliphatic acyl group may be linear or branched, and may be saturated or unsaturated. Meanwhile, m is an integer of 0 to 8, and X represents at least one of ethylene, propylene, and butylene, and n is an integer of 1 to 1,000. R⁸ is a hydrogen atom or a 2-decenoyl group. R⁷ and R⁸ are independent each other.
Meanwhile, a feed additive of the present invention may comprise a kind of rhamnolipid or plural kinds of rhamnolipids.

Rhamnolipids to be used in the present invention can be obtained by culturing a bacterium. For example, there may be used bacteria belonging to the genera *Pseudomonas* and *Burkholderia.* Of those, bacteria belonging to the genus *Pseudomonas* are preferably used. Examples of the bacteria belonging to the genus *Pseudomonas* include *Pseudomonas aeruginosa and Pseudomonas chlororaphis,* and *Pseudomonas sp.* may be used. Examples of the bacteria belonging to the genus *Burkholderia* include *Burkholderia pseudomalle.* Of those, *Pseudomonas aeruginosa* is particularly preferably used. Specifically, for example, *Pseudomonas aeruginosa* NBRC 3924 strain, *Pseudomonas sp.* DSM 2874 strain, etc. may be used.
NBRC 3924 strain is registered in the department of NITE Biological Resource Center (NBRC) in National Institute of Technology and Evaluation.
DSM 2874 strain is registered in Deutsche Sammlung von Mikroorganismen und Zellkulturen GmbH (DSMZ).
Meanwhile, the rhamnolipids may be synthesized or may be a commercially available product.

The MEL and rhamnolipids can be produced using the above-mentioned microorganisms by the following method.
MEL can be produced by: selecting a material suitable for a bacterium to be used from natural oils and fats, fatty acids, alcohols, ketones, hydrocarbons, n-alkanes, etc.; and culturing the bacterium at a culture temperature generally used for culture of the bacterium. The natural oils and fats are preferable materials, and for example, soybean oil, sunflower oil, coconut oil, cottonseed oil, corn oil, palm oil, etc. may be used, and of those, soybean oil is particularly preferably used.
On the other hand, rhamnolipids can be produced by: selecting a material suitable for a bacterium to be used from natural oils and fats, fatty acids, alcohols, ketones, hydrocarbons, n-alkanes, sugars, etc.; and culturing the bacterium at a culture temperature generally used for culture of the bacterium. As such a method, for example, the method described in JP 10-75796 A may be used.
In each case, the culture method is not particularly limited, and examples thereof include a liquid culture method and a solid culture method by static culture, reciprocal shaking culture, rotary shaking culture, jar-fermenter culture.

Production of MEL using yeast belonging to the genus *Pseudozyma* can be performed by adding a natural oil and fat such as soybean oil to a medium generally used for culture of yeast belonging to the genus *Pseudozyma* and culturing the bacterium at 20°C to 35°C.
Production of rhamnolipids using a bacterium belonging to the genus *Pseudomonas* can be performed by adding a natural oil and fat such as soybean oil, a sugar such as glucose, and an alcohol such as ethanol to a medium generally used for culture of a bacterium belonging to the genus *Pseudomonas* and culturing the bacterium at 20°C to 40°C.

In production of MEL and/or rhamnolipids using a microorganism, there may be used a purified product of MEL and/or rhamnolipids obtained by purifying a culture product, or a fraction containing MEL and/or rhamnolipids obtained by centrifuging a culture product. Meanwhile, a culture product may be used as it is, and for example, a product obtained by drying/pulverizing a culture solution or a solid culture product may be used.

A feed additive of the present invention may contain either or both of MEL and rhamnolipids . Although the MEL and/or rhamnolipids content is not particularly limited, from the viewpoint of achieving a sufficient effect, it is preferably 10 ppm by mass or more, more preferably 1% by mass or more.

In addition, a feed additive of the present invention may further contain not only MEL and/or rhamnolipids but also an arbitrary component such as a component effective for prevention or treatment of diseases of birds or mammals, a component effective for promotion of growth of ruminant animals, a nutrition supplement component, or a component for enhancement of preservation stability. Examples of the arbitrary component include: viable cell agents of *Enterococci, Bacilli,* and *Bifidobacteria;* enzymes such as amylase and lipase; vitamins such as L-ascorbic acid, choline chloride, inositol, and folic acid; minerals such as potassium chloride, ferric citrate, magnesium oxide, and phosphate and amino acids such as DL-alanine, DL-methionine, and L-lysine hydrochloride; organic acids such as fumaric acid, butyric acid, lactic acid, acetic acid, and salts thereof; antioxidants such as ethoxyquin and dibutylhydroxytoluene; fungicides such as calcium propionate; binders such as CMC, sodium casein, and sodium polyacrylate; emulsifers such as glycerine fatty acid ester and sorbitan fatty acid ester; pigments such as astaxanthin and canthaxanthin; and flavors such as various esters, ethers, and ketones.

The dosage form of a feed additive of the present invention is not particularly limited, and the feed additive may have any form such as powder, liquid, or tablet. A feed additive of the present invention can be produced by: mixing MEL and/or rhamnolipids, and an optional component, if necessary; and formulating the mixture.

Meanwhile, MEL and rhamnolipids show antibiotic activities to bacteria that cause diseases of birds or mammals, therefore, a feed additive of the present invention can be used for preventing or treating those diseases of birds or mammals caused by the bacteria.
A feed additive of the present invention can be preferably used for preventing or treating, in particular, an infectious disease caused by a Gram-positive bacterium.
Examples of the gram-positive bacteria include bacteria belonging to genuses of *Micrococcus, Staphylococcus, Streptococcus. Planococcus, Stomatococcus, Enterococcus, Peptococcus, Peptostreptococcus, Ruminococcus, Leuconostoc, Pediococcus, Aerococcus , Gemella, Coprococcus, Sarcina, Bacillus, Clostridium, Lactobacillus, Listeria, Erysipelothrix. Corynebacterium, Rhodococcus, Propionibacterium, Eubacterium, Actinomyces, Bifidobacterirum, Mycobacterium, Nocardia,* and *Dermatophilus.*
The feed additive of the present invention can be suitably used for prevention and treatment of disease induced by bacteria belonging to the genuses of *Staphylococcus* and *Streptococcus,* and, specifically, by *Staphylococcus aureus, Staphylococcus epidermidis, Streptococcus suis, Streptococcus bovis* and the like.

A feed for birds or mammals can be obtained by mixing a feed additive of the present invention with another feed component to be used in feeds for birds or mammals, pet foods, supplements for pets (hereinafter, referred to as feed). The type and components of the feed are not particularly limited. Meanwhile, the above-mentioned arbitrary component which can be added to a feed additive may be added to a feed of the present invention. In addition, a feed of the present invention may be used as a feed to be used for preventing or treating diseases of birds or mammals.

The MEL and/or rhamnolipids content in a feed of the present invention is not particularly limited, it is appropriately adjusted depending on the species of a target animal, physical condition, type of a feed, feed component, age, sex, weight, etc. The content of the MEL and/or rhamnolipids is preferably 1 to 10,000 ppm by mass, more preferably 10 to 10,000 ppm by mass, further preferably 10 to 1,000 ppm by mass per dry mass.

A feed of the present invention can be produced by adding a feed additive to a feed component as it is and mixing the components. In this procedure, in the case of using a powder feed additive or a solid feed additive, the form of the feed additive may be changed into a liquid or gel in order to mix the components easily. In this case, water; plant oils such as a soybean oil, a rapeseed oil, a corn oil; a liquid animal oil; or water-soluble polymer components such as polyvinylalcohol, polyvinylpyrrolidone, or polyacrylic acid may be used as a liquid carrier. Meanwhile, in order to keep uniformity of MEL and/or rhamnolipids in a feed, it is preferable to incorporate alginic acid, sodium alginate, xanthan gum, casein sodium, gum Arabic, guar gum, or a water-soluble polysaccharide such as a tamarind seed polysaccharide.

The species of animals that eat a feed of the present invention are birds or mammals. The feed may be used for livestock or pets such as dogs and cats, for example. Of those, the feed is preferably used for breeding livestock, in particular, chickens, pigs, or cows. The feed is preferably used for breeding ruminant animals. For example, the feed is preferably used for breeding cow, goat, and sheep. The amount of the feed to be given may be appropriately adjusted depending on the animal's species, weight, age, sex, physical condition, feed component, etc.
The method of giving a feed and method of breeding an animal may be methods generally used depending on the species of the animal.

### [Examples]

### [I] Evaluation of antibiotic activities

### <1> Production of MEL

### (1) Culture of Pseudozyma yeast

### (Preculture)

10 ml of potato dextrose medium was added to a test tube, and a silicone plug was inserted therein. The tube was sterilized in an autoclave, and *Pseudozyma aphidis* NBRC 10182 was inoculated, followed by shaking culture at 30°C for 24 hours.

### (Main culture)

50 ml of a medium containing ion-exchanged water, 8% soybean oil, 0.2% NaNO₃, 0.02% KH₂PO₄, 0.02% MgSO₄·7H₂O, and 0.1% yeast extract were added to a 500-ml Erlenmeyer flask, and a silicone plug was inserted therein, followed by sterilization in an autoclave. The above-mentioned NBRC 10182 preculture solution was added thereto, and shaking culture was performed at 30°C/220 rpm for 7 days.

### (2) Extraction/purification of MEL

### (Extraction)

50 ml of the culture solution obtained in the main culture was dispensed into a separating funnel, and extraction was performed twice with an equal amount of ethyl acetate. The ethyl acetate layers were combined, and the solvent was distilled off. Thereafter, the residue was dissolved in 25 ml of methanol and washed twice with 50 ml of hexane, and methanol was distilled off, to thereby yield a crude purified product of MEL (purity 69%, determined by anthrone reaction described below).

### (Purification)

1 g of the above-mentioned crude purified product was dissolved in a small amount of chloroform, and fractionation was performed using a silica gel column. 500 ml of chloroform, 500 ml of chloroform/ethyl acetate = 4/1, 500 ml of acetone, and 500 ml of methanol were sequentially passed through the column to perform fractionation.

The resultant fractions were developed by thin-layer chromatography (developing solvent CHCl₃/MeOH/water = 65/15/2), and fractions having the Rf values of the respective MEL described in the following document 1) (Rf = 0.52, 0.58, 0.63, 0.77) were selected and combined, to thereby yield a standard sample.

### 1) Agric. Biol. Chem., 54(1) 31-36, 1990

### (Purity determination: anthrone reaction)

The crude purified product diluted to an appropriate concentration with ethyl acetate was added to a test tube, and the solvent was distilled off. To the test tube was added 5 ml of an anthrone reagent (0.2% anthrone 75% sulfuric acid), and the mixture was allowed to react in boiling water for 10 minutes, followed by measurement of an absorbance at 620 nm. The purity of the crude purified product was calculated by comparing the absorbance with that of the standard sample.

### <2> Production of rhamnolipids

### (1) Culture of Pseudomonas bacterium

### (Preculture)

10 ml of peptone medium was added to a test tube, and a silicone plug was inserted therein. The tube was sterilized in an autoclave, and *Pseudomonas aeruginosa* NBRC 3924 was inoculated, followed by shaking culture at 30°C for 24 hours.

### (Main culture)

50 ml of a medium containing ion-exchanged water, 0.2% CaCO₃, 0.05% K₂HPO₄, 0.05% MgSO₄·7H₂O, 0.5% yeast extract, and 0.5% Soy flour were added to a 500-ml Erlenmeyer flask, and a silicone plug was inserted therein, followed by sterilization in an autoclave. To the flask were added 1 ml of filter-sterilized ethanol and the above-mentioned NBRC 3924 preculture solution, and 0.75 ml of filter-sterilized ethanol was added every two days, followed by shaking culture at 28°C/220 rpm for 8 days.

### (2) Extraction/purification of rhamnolipids

### (Extraction)

50 ml of the culture solution obtained in the main culture was dispensed into a separating funnel, and extraction was performed twice with methanol/chloroform = 1/1. Organic layers were combined, and the solvent was distilled off, to thereby yield a crude purified product of rhamnolipids (purity 55%, determined by anthrone reaction described below).

### (Purification)

450 ml of the culture solution obtained in the above-mentioned main culture was adjusted to pH 3, and the bacterial cells were removed by centrifugation. The supernatant was passed through a column filled with TSK gel DEAE-TOYOPEARL 650 M and previously treated with 0.5 M Tris-HCl buffer (pH 9.0), and the column was washed with 0.5 M Tris-HCl buffer (pH 9.0). Then, 0.5 M Tris-HCl buffer (pH 9.0) with NaCl with concentrations of 0 to 0.4 M was passed through the column in a gradient manner at 2.3 ml/min to elute and fractionate rhamnolipids captured in the gel.

The respective fractions were developed by thin-layer chromatography (developing solvent CHCl₃/MeOH/water = 65/25/4), and fractions having Rf values of rhamnolipids (Rf = 0.32, 0.52) described in the following document 2) were selected, followed by extraction with methanol/chloroform = 1/1. The fractions were combined, and the solvent was distilled off, to thereby yield a standard sample.

### 2) Biotechnology Letters, 54(12) 1213-1215, 1997

### (Purity determination: anthrone reaction)

The crude product diluted to an appropriate concentration with methanol was added to a test tube, and the solvent was distilled off. To the test tube was added 5 ml of an anthrone reagent (0.2% anthrone 75% sulfuric acid), and the mixture was allowed to react in boiling water for 10 minutes, followed by measurement of an absorbance at 620 nm. The purity of the crude purified product was calculated by comparing the absorbance with that of the standard sample.

### <3> Evaluation of antibiotic activities

Minimum inhibitory concentrations (MICs) of each of the bacteria shown in Table 1 were measured for the MEL and rhamnolipids by the following procedures.

The above-mentioned bacteria were precultured in a bouillon medium for sensitivity determination (NISSUI PHARMACEUTICAL CO. , LTD.). The concentrations of the bacteria in the culture solutions were adjusted to about 1.0 × 10⁵ to 10⁶ CFU/ml with physiological saline, and the bacteria were inoculated into the measurement mediums. As the measurement mediums, a medium for sensitivity measurement (NISSUI PHARMACEUTICAL CO. , LTD.) was used for *Staphylococcus aureus, Staphylococcus epidermidis,* and *Bacillus subtilis,* and a blood agar medium (heart infusion medium: NISSUI PHARMACEUTICAL CO., LTD., sheep sterile defibrinated blood: Kohjin Bio Co. Ltd.) was used for *Streptococcus suis* and *Streptococcus bovis.* Culture was performed at 37° C for about 20 hours under aerobic conditions for *Staphylococcus aureus, Staphylococcus epidermidis,* and *Bacillus subtilis* or under 5% CO₂ conditions for *Streptococcus suis* and *Streptococcus bovis.* After completion of culture, the MICs were measured.

The MEL crude purified product obtained in section <1> (purity 69%) was used as MEL, and the rhamnolipid crude purified product obtained in section <2> (purity 55%) was used as rhamnolipids. Meanwhile, for the purpose of comparison, MICs of sucrose ester (sucrose manodecanoate: manufactured by SIGMA-ALDRICH Japan K.K.), mannose (manufactured by Wako Pure Chemical Industries, Ltd.), and rhamnose (manufactured by SIGMA-ALDRICH Japan K.K.) were measured.
The results are shown in Table 1.

**Table 1**

| Bacteria | MEL | Rhamnolipids | Sucrose ester | Mannose | Rhamnose |
|---|---|---|---|---|---|
| Staphylococcus aureus | 50 | 12.5 | <1600 | <1600 | <1600 |
| Staphylococcus epidermidis | 25 | 12.5 | <1600 | <1600 | <1600 |
| Streptococcus suis | 50 | 50 | 800 | <1600 | <1600 |
| Streptococcus bovis | 50 | 12.5 | 800 | <1600 | <1600 |
| Bacillus subtilis | 12.5 | 6.25 | 400 | <1600 | <1600 |

The MEL and rhamnolipids were found to have antibiotic activities to the *Staphylococcus, Streptococcus,* and *Bacillus* bacteria several times or dozens of times higher than the antibiotic activity of sucrose manodecanoate that is a glycolipid. On the other hand, mannose and rhamnose that are components of a glycolipid were found to have no antibiotic activities.
Therefore, if birds or mammals are fed with MEL and/or rhamnolipids, diseases caused by the above-mentioned bacteria will be prevented or treated.

### [II] Evaluation of generation amounts of gas and volatile fatty acid

### <1> Production of mannosylerythritol lipids (MEL)

### (1) Culture of Pseudozyma yeast

### (Preculture)

10 ml of potato dextrose medium was added to a test tube, and a silicone plug was inserted therein. The tube was sterilized in an autoclave, and *Pseudozyma aphidis* NBRC 10182 was inoculated, followed by shaking culture at 30°C for 24 hours.

### (Main culture)

50 ml of a medium containing ion-exchanged water, 8% soybean oil, 0.2% NaNO₃, 0.02% KH₂PO₄, 0.02% MgSO₄·7H₂O, and 0.1% yeast extract were added to a 500-ml Erlenmeyer flask, and a silicone plug was inserted therein, followed by sterilization in an autoclave. The above-mentioned NBRC 10182 preculture solution was added thereto, and shaking culture was performed at 30°C/220 rpm for 10 days.

### (2) Purification of MEL

### (Purification)

50 ml of the above-mentioned culture solution was adjusted to pH 3 with 1N HCl, and the supernatant was removed by centrifugation. 50 ml of pure water was added to the precipitates, and centrifugation was performed again to recover the precipitates. The precipitates were dissolved in 10 ml MeOH, and 10 ml of hexane was further added to wash the precipitates (three times). Then, 10 ml of water was added to the MeOH solution after washing, and MEL were extracted with 10 ml of chloroform from the solution (three times). The chloroform layers were combined, and the solvent was distilled off, to thereby yield a crude purified product. The purity was found to be 90% by anthrone reaction.

### (Standard sample)

1 g of the above-mentioned crude purified product was dissolved in a small amount of chloroform, and fractionation was performed using a silica gel column. 500 ml of chloroform, 500 ml of chloroform/ethyl acetate = 4/1, 500 ml of acetone, and 500 ml of methanol were sequentially passed through the column to perform fractionation.
The resultant fractions were developed by thin-layer chromatography (developing solvent CHCl₃/MeOH/water = 65/15/2), and fractions having the Rf values described in Agric. Biol. Chem., 54(1), 31-36, 1990 (Rf values of the respective MEL, Rf = 0.52, 0.58, 0.63, 0.77) were selected and combined, to thereby yield a standard sample.

### (Purity determination: anthrone reaction)

The partially product diluted to an appropriate concentration with ethyl acetate was added to a test tube, and the solvent was distilled off. To the test tube was added 5 ml of an anthrone reagent (0.2% anthrone 75% sulfuric acid), and the mixture was allowed to react in boiling water for 10 minutes, followed by measurement of an absorbance at 620 nm. The purity of the crude purified product was calculated by comparing the absorbance with that of the standard sample.

### <2> Rhamnolipids (RL)

BFL Biosurfactant (rhamnolipids) manufactured by Bio Future Ltd. was dried and used.

### <3> Effect of rhamnolipids (RL) and mannosylerythritol lipids (MEL) on gas generation and volatile fatty acid generation in rumen (1) Sample

The above-mentioned rhamnolipids (RL) and mannosylerythritol lipids (MEL) were used for a test. As a culture inoculum, there was used a rumen fluid (filtered through four layers of gauze) collected from a Holstein female cow (fitted with a rumen cannula) of Experiment Farm, Field Science Center for Northern Biosphere, Hokkaido University. The inoculum was diluted 2-fold with McDougal's artificial saliva (pH 6.8) and used.

### (2) Culture

Culture was performed in test culture solutions with RL content of 500 µg/ml and with MEL content of 500 µg/ml. 0.05 g of each of RL and MEL were dissolved in 1 ml ethanol, and 100 µl of each solution was added to a Hungate tube. The solutions were allowed to stand for several hours to volatilize ethanol. To the tubes were added 0.15 g of cornstarch, 0.025 g of mixed feed powder, and 0.025 g of Orchard grass dry powder as culture substrates. The above-mentioned diluted rumen fluid was added in an amount of 10 ml, and butyl rubber caps and plastic screw caps were inserted in the tubes while nitrogen gas was supplied to their headspaces, followed by anaerobic culture in a water bath (37°C, 18 hours).

No reagent (only ethanol: control group), RL (RL group), or MEL (MEL group) was added to treat each of the culture solutions, and culture was performed in quintuplicate.

### (3) Analysis

Methane, hydrogen, and carbon dioxide were analyzed by TCD gas chromatography. Total volatile fatty acid (VFA) concentrations and compositions were measured by FID gas chromatography.

### (4) Results

### (i) Gas generation

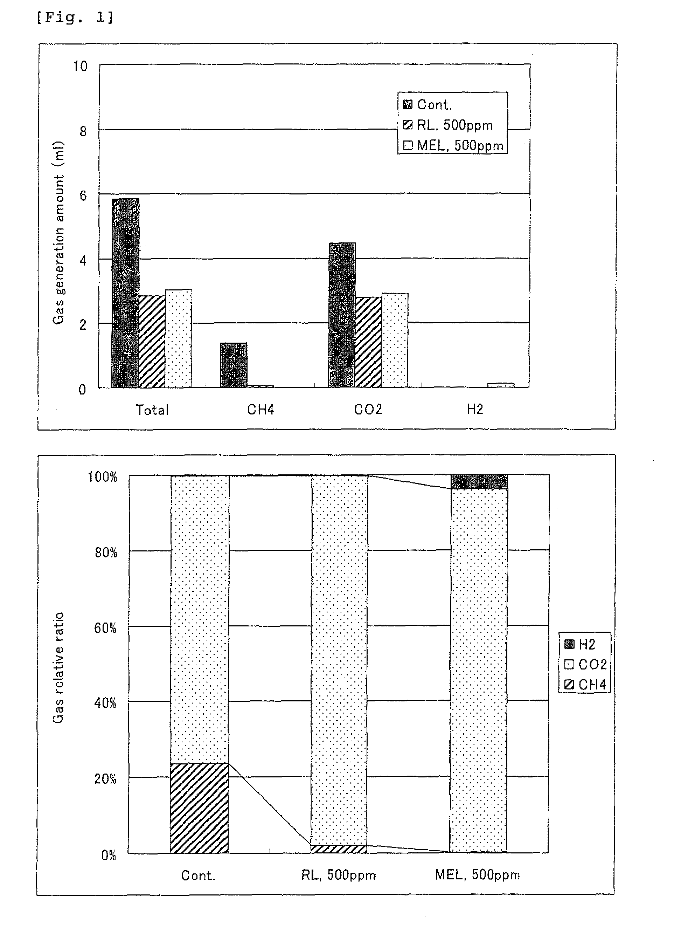

The total gas amounts for 18 hours after initiation of culture were found to decrease both in the cases of the RL group and MEL group (decreased by 51% and 48%, respectively). In particular, decreases in methane amounts were significantly high, and the methane amounts of the RL group and MEL group were decreased by 96% and 99%, respectively, which revealed that almost all the methane generation disappeared. The carbon dioxide amounts of the RL group and MEL group were found to decrease by 37% and 35%, respectively. The ratios of methane to the total gas of the RL group and MEL group were found to decrease to 2.1% and 0.3%, respectively, compared with the control group (23.7%).
The results are shown in Table 2 and Figs. 1.

**Table 2**

| Analysis items | | Test | | | | | |
|---|---|---|---|---|---|---|---|
| | | Cont. | | RL500 | | MEL500 | |
| Gas generation amount (ml) | | | | | | | |
| | Total | 5.86±1.51 | a | *2.86±0.22* | b | *3.04±0.39* | b |
| | CH4 | 1.39±0.58 | a | *0.06±0.02* | b | *0.01±0.00* | c |
| | CO2 | 4.46±0.93 | a | *2.79±0.22* | b | *2.92±0.37* | b |
| | H2 | 0.01±0.00 | a | 0.01±0.00 | a | *0.12±0.02* | b |
| Gas relative ratio (%) | | | | | | | |
| | CH4 | 23.7±5.9 | a | *2.1±0.5* | b | *0.3±0.1* | c |
| | CO2 | 76.1±5.9 | a | *97.6±0.5* | c | *96.0±0.3* | b |
| | H2 | 0.16:±0.03 | a | *0.28±0.01* | b | *3.77±0.27* | c |

| | | | | | | | |
|---|---|---|---|---|---|---|---|
| a, b, c: There are significant differences among different symbols. There is a significant difference between b and c similar to that between a and b. Italic: There is a significant difference compared with control. | | | | | | | |

### (ii) Generation of volatile fatty acid (VFA)

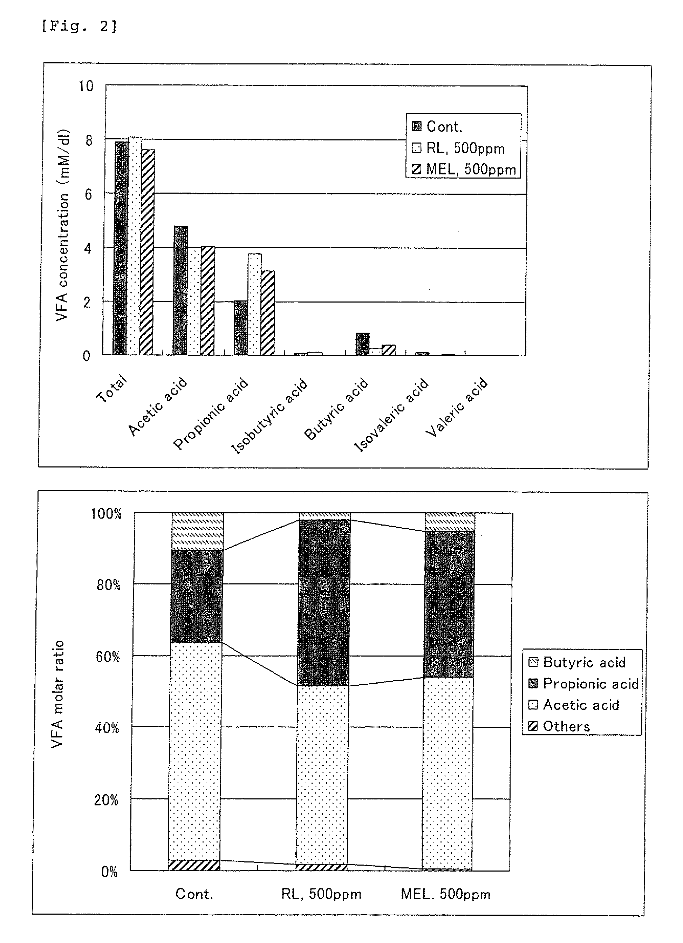

The total VFA concentrations were not affected by the treatments, but the VFA production pattern for each case was drastically altered. That is, the concentrations of acetic acid, butyric acid, isobutyric acid, valeric acid, and isovaleric acid were found to significantly decrease by adding RL and MEL. On the other hand, the concentration of propionic acid was found to significantly increase (increased by 85% (RL group) and by 53% (MEL group)). The molar ratios of the respective acids were found to increase in the case of propionic acid (from 25.8% to 46.7% and 41.1%) or decrease in the cases of acetic acid and butyric acid (from 60.9% to 49.7% and 53.4%, and from 10.6% to 2.0% and 5.0%, respectively), and all the differences were significant. In particular, the ratio of propionic acid increased to a level higher than that in general rumen.
The results are shown in Table 3 and Fig. 2.

**Table 3**

| Analysis items | | Test | | | | | |
|---|---|---|---|---|---|---|---|
| | | Cont. | | RL500 | | MEL500 | |
| VFA concentration (mM/dl) | | | | | | | |
| | Total | 7.87±0.65 | | 8.06±0.34 | | 7.61±0.68 | |
| | Acetic acid | 4.79±0.36 | a | *4.00±0.12* | b | *4.05±0.20* | b |
| | Propionic acid | 2.04±0.28 | a | *3.77±0.24* | b | *3.13±0.38* | b |
| | Isobutyric acid | 0.09±0.01 | a | 0.11±0.12 | a | *0* | b |
| | Butyric acid | 0.83±0.05 | a | *0.27±0.01* | c | *0.38±0.06* | b |
| | Isovaleric acid | 0.12±0.02 | a | *0.01±0.03* | b | *0.04±0.04* | b |
| | Valeric acid | 0 | | 0 | | 0 | |
| VFA molar ratio (%) | | | | | | | |
| | Acetic acid | 60.9±0.8 | a | *49.7±0.7* | b | *53.4±2.2* | b |
| | Propionic acid | 25.8±1.7 | a | *46.7±1.2* | c | *41.1±1.4* | b |
| | | | | | | | |
| | Butyric acid | 10.6±0.8 | a | *2.0±1.8* | c | *5.0±0.4* | b |

| | | | | | | | |
|---|---|---|---|---|---|---|---|
| a, b, c: There are significant differences among different symbols. There is a significant difference between b and c similar to that between a and b. Italic: There is a significant difference compared with control. | | | | | | | |

### [Industrial Applicability]

If the feed additive of the present invention is mixed in the feed and given to birds or mammals, diseases can be prevented or treated. Specifically, the feed of the present invention can prevent or treat an infectious disease caused by a Gram-positive bacterium. The feed containing the feed additive of the present invention can be suitably used for breeding livestock such as chickens, pigs, and cows. In addition, the feed additive of the present invention has high biodegradability and is very safe for living beings and environment.
Meanwhile, if the feed additive of the present invention is mixed in the feed and given to ruminant animals, it is possible to suppress generation of methane and to promote generation of propionic acid, resulting in promotion of growth of the ruminant animals and improvement of the feed efficiency. The feed containing the feed additive of the present invention can be suitably used for breeding ruminant animals such as cow, goat, and sheep. In addition, the feed additive of the present invention has high biodegradability and is very safe for living beings and environment.

## Claims

1. A feed additive for birds and mammals, comprising mannosylerythritol lipids and/or rhamnolipids.

2. The feed additive according to Claim 1, which is for livestock.

3. The feed additive according to Claim 2, wherein the livestock is a chicken, pig, or cow.

4. The feed additive according to Claim 1, which is for a ruminant animal.

5. The feed additive according to any one of Claims 1 to 4, wherein the mannosylerythritol lipids are obtained from a yeast belonging to the genus *Pseudozyma.*

6. The feed additive according to any one of Claims 1 to 5, wherein the rhamnolipids are obtained from a bacterium belonging to the genus *Pseudomonas.*

7. The feed additive according to any one of Claims 1 to 6, which is for prevention or treatment of a disease.

8. The feed additive according to Claim 7, wherein the disease is an infectious disease caused by a Gram-positive bacterium.

9. The feed additive according to Claim 8, wherein the Gram-positive bacterium is a bacterium belonging to the genus *Staphylococcus* or *Streptococcus.*

10. The feed additive according to Claim 9, wherein the Gram-positive bacterium is *Staphylococcus* aureus, *Staphylococcus epidermidis, Streptococcus suis,* or *Streptococcus bovis.*

11. A feed comprising the feed additive according to any one of Claims 1 to 10.

12. A method of breeding birds or mammals, comprising giving the feed according to Claim 11 to birds or mammals.
